Europäisches Patentamt

European Patent Office

Office européen des brevets

(11) Publication number: **0 362 998**
**A2**

# EUROPEAN PATENT APPLICATION

(21) Application number: 89308483.0

(22) Date of filing: 22.08.89

(51) Int. Cl.5: **G01N 33/569** , **G01N 33/577** , **C12P 21/08**

(30) Priority: 07.09.88 US 241907

(43) Date of publication of application:
**11.04.90 Bulletin 90/15**

(84) Designated Contracting States:
**CH DE ES FR GB IT LI NL SE**

(71) Applicant: **MINNESOTA MINING AND MANUFACTURING COMPANY**
**3M Center, P.O. Box 33427**
**Saint Paul, MN 55133- 3427(US)**

(72) Inventor: **Shelburne, Charles E. c/o Minnesota Mining and**
**Manufacturing Company 2501 Hudson Road**
**St-Paul Minnesota 55133-3427(US)**

(74) Representative: **Baillie, Iain Cameron et al**
**c/o Ladas & Parry Isartorplatz 5**
**D-8000 München 2(DE)**

(54) Monoclonal antibodies specific for eikenella corrodens.

(57) A method for determining the presence and/or amount of Eikenella corrodens in an oral sample such as gingival and subgingival plaque. Using species-specific antibodies specific for the entire species E. corrodens, the presence and/or amount of complex formed with antigens of E. corrodens in the sample is determined. Species-specific antibodies, and kits containing such antibodies, can include monoclonal antibodies.

EP 0 362 998 A2

# MONOCLONAL ANTIBODIES SPECIFIC FOR EIKENELLA CORRODENS

## TECHNICAL FIELD

The present invention relates to monoclonal antibodies and antigens, and to the use of such antibodies and antigens for the detection of the presence and concentration of microorganisms implicated in the etiology of periodontal disease. More specifically, the present invention relates to antibodies specific to antigens from Eikenella corrodens.

## BACKGROUND ART

Clinical assays specific to microorganisms in gingival and subgingival plaque are useful in the diagnosis of periodontal disease, in evaluating the progress of periodontal therapy, and in determining the status of a patient at subsequent recall examinations. The standard microbiological techniques are time consuming, expensive, and require a high level of expertise. Further, such tests frequently give results that are not as accurate or as sensitive as desired or required.

Recently, various efforts have been made to improve or replace standard microbiological techniques by the use of immunodiagnostic assay techniques. Such immunodiagnostic techniques are based upon the formation of a complex between the antigen being assayed and an antibody or antibodies, in which one or the other member of the complex is generally either labeled or is capable of being labeled, in order to allow the amount of complex formed to be determined. Although immunodiagnostic techniques can be a substantial improvement over previously used detection techniques, further improvements remain to be made, particularly with respect to the specificity of the antibodies to be used.

Eikenella corrodens is an oral gram-negative facultative organism that is believed to be associated with severe oral and non-oral infections. Its primary oral ecological niche appears to be dental plaque and periodontal pockets. See, e.g., Maia, et al., J. Inf. 2:347-353 (1980).

Antigens specific for one or more strains or serotypes of E. corrodens but less than the entire known species of E. corrodens, have been described, for example, in the article of Maliszewski, et al., Inf. Immun. 42:208-213 (1983), which also describes monoclonal antibodies for such type-specific antigens. A monoclonal antibody to a type-specific antigen would clearly not alone be suitable as a diagnostic reagent for determining the presence of all types of the species E. corrodens.

Anti-Eikenella antisera has been described for use in serological testing of E. corrodens. See, e.g., Badger, et al., Int'l. J. Syst. Bacteriol. 31:446 (1981) and Tanner, et al., J. Perio. Res. 22:327-330 (1987).

Considering the relatedness of E. corrodens to many other oral species, and the potential role of this species in periodontal disease, what is needed is a method of identifying and differentiating E. corrodens, as a species, in the complex microbiological and chemical environment that typifies dental plaque.

## SUMMARY OF THE INVENTION

The present invention provides a novel method of determining the presence and/or quantitating the amount of Eikenella corrodens in an oral sample comprising the steps of:

(a) contacting the oral sample with an antibody specific to the species E. corrodens in order to form an immunological complex between antigens of E. corrodens present in the sample and the antibody, and

(b) determining the amount of complex formed.

The present invention provides a species-specific antibody showing specificity for E. corrodens and showing substantially no cross-reactivity with normal human antigens. The present antibodies are typically employed as reagents in an immunoassay format useful for detecting the presence and amounts of E. corrodens in a clinical oral sample such as plaque, or gingival or subgingival fluid. Such immunoassays generally involve the steps of contacting the sample with an amount of the species-specific antibody for a time sufficient to allow antibody/antigen complex to form, and subsequently determining the amount of such complex formed.

The method of the present invention allows the detection of E. corrodens in an oral sample, such as plaque, or gingival or subgingival fluid, in a manner that is rapid, precise, simple, and yields significant and useful results, i.e., the results correlate well with results obtained by the longer and more laborious method of anaerobic cultivation of the microbes. By employing antibodies, this method offers significant advantages over more traditional approaches, such as those requiring cultivation, staining, or otherwise identifying such microbes through laborious or expensive techniques.

## DETAILED DESCRIPTION

The word "amount" as used herein refers to a determination that is sufficiently quantitative and/or semiquantitative for its intended purpose, e.g., quantitative as in the determination of the actual number or concentration of complex, and in turn the number or concentration of cells and/or antigen in the oral sample, or semiquantitative as in the sense of a comparative determination showing the amount of complex formed, and in turn the amount of cells and/or antigen, as being at a level above or below a predetermined level (e.g., a "yes or no" test).

The word "antibody" as used herein refers to any antibody or portion thereof that is sufficiently discriminating to enable its use in the method of the invention, e.g., monospecific polyclonal antibodies as well as monoclonal antibodies. Preferred antibodies for use in the method of the present invention are monoclonal antibodies.

While any immunoglobulin can be employed, IgG is preferred. Either whole antibodies or fragments thereof can be employed. Single monoclonal antibodies can be employed, or mixtures thereof, including mixtures of monoclonal antibodies or mixtures of polyclonal antibodies. The number and type of antibodies that are employed will depend upon the antigenic site(s) and number of different antigens that are to be detected. The antibody composition preferably is free of non-specific antibodies, i.e., antibodies that bind to antigens other than the desired antigens.

The antibodies useful in the present invention can be obtained by a variety of methods within the ability of those skilled in the art, including in vitro stimulation of lymphocytes with mitogens and/or antigens, splenic fragment culture, virus transformation of lymphocyte clones, or recombinant DNA techniques.

Monoclonal antibodies are preferably obtained by a process for the production of hybridomas similar to that discussed by Milstein and Kohler and reported in Nature, 256:495-497 (1975), the disclosure of which is hereby incorporated by reference. This process involves injecting a mouse (or other suitable animal) with the desired immunogenic material. In the present invention that material can be a culture of partially or completely purified E. corrodens. That material can also be a purified or partially purified component of E. corrodens, such as enzymes or toxins, or lipopolysaccharide (LPS) which has been purified, e.g., by the sonication and extraction method described in WO84/04458, or other methods cited therein, the disclosure of which is hereby incorporated by reference.

To prepare monoclonal antibodies, suitable hosts such as mice are immunized with potential antigen-containing suspensions of cells or cell fractions according to immunization methods, routes, and schedules generally in keeping with established techniques for antibody stimulation and production. After immunization the immune lymphoid cells are isolated, preferably from the spleens, and are fused with suitable myeloma, plasmacytoma, or hybridoma cells to generate hybrid cell lines (i.e., hybridomas) that produce monoclonal antibodies to the antigens of choice and that can be cultivated and subcultivated indefinitely.

The population of hybridomas formed by fusion can then be screened for immunoglobulin production. Any of the several known methods for screening for immunoglobulins can be used, such as enzyme-linked immunoassay (ELISA) using purified antigen, according to known techniques. The immunoglobulins present in the cell culture fluids are further examined for their ability to react with the microbial cells or fractions used for immunization. This can be accomplished by modifying the above-mentioned immunoassay according to methods known to the art.

If hybridoma cultures are found to produce monoclonal antibodies that react with the immunizing antigen they are further examined to determine their specificity. The cultures are cloned by limiting dilution to assure monoclonality using techniques within the skill of those skilled in the art, e.g., Thomas J. McKearn, "Cloning of Hybridoma Cells by Limiting Dilution in the Fluid Phase" in Monoclonal Antibodies, p. 374, Kennett, R. H., McKearn, T. J. and K. B. Bechtol, eds. Plenum Press, N.Y. (1980).

The monoclonal antibodies produced by these cloned hybridomas are tested to determine their cross-reactivity with antigens other than those of the immunizing species, strain, or antigen by an ELISA assay. In addition, the antibodies can be assayed for their ability to bind microbial cell components separated by electrophoretic means (e.g., "Western Blots" and immunoprecipitation) known to those skilled in the art (see

3

e.g., Tobin, et al., Proc. Nat. Acad. Sci. 76:4350-4354 (1979)).

If any hybridoma cell lines are found to produce monoclonal antibodies with the desired specificity for the species E. corrodens, they can then be cultivated and subcultivated to establish continuous production of antibodies, e.g., by tissue culture of mouse ascites fluid production techniques conventional in the art. These cell lines can be stored and preserved by conventional techniques, including freezing.

The antibodies used in the assay are preferably isolated by conventional techniques such as ion exchange chromatography or precipitation, e.g., by treating tissue culture fluid or clarified ascites fluid with 50% ammonium sulfate. This treatment results in the precipitation of antibodies. The precipitate is optionally, and preferably, resuspended in a buffered saline solution (phosphate buffered saline, "PBS", pH 7.5) for further use. Recovered antibodies can be stored, e.g., lyophilized, frozen or refrigerated according to known techniques.

Antigens suitable for use in the method of the present invention include any cellular or extracellular molecular species that allow one to detect the species E. corrodens without substantial cross-reactivity with any other oral periodontal microbial antigens or human antigens.

Cellular components or products suitable for providing antigenic sites (i.e., epitopes) for such purposes include any that are capable of serving as antigens, i.e., are immunogenic alone or as haptens, and that are capable of being immunologically reactive with monoclonal antibodies in oral samples evaluated according to the method of the invention.

Suitable antigens include those located in, e.g., the cell envelope, capsule, or cytoplasm, or antigens produced by E. corrodens and found in its extracellular environment such as extracellular factors, toxins, or enzymes.

Preferred antigens are those showing optimal specificity and binding characteristics for E. corrodens, as well as those antigens that are detectable with minimal physical manipulation of the sample or cell, e.g., extracellular or easily accessible cell wall or envelope antigens.

A preferred antigenic source is LPS. For a review of LPS see, generally, R.J. Elin, et al., CRC Handbook of Microbiology, Laskin, et al., eds., Vol. II, 215-239 (1973), and I.W. Sutherland, Ann. Rev. Microbiol. 39:243-270 (1985).

A number of articles have described the biological activity, composition, and antigen structure of the LPS of E. corrodens, see e.g., respectively (biological activity) Behling, et al., Inf. Imm. 26(2):580-584 (1979), Progulske, et al., Inf. Imm. 43(1):178-182 (1984), and Okuda, et al., Inf. Imm. 55(12):3192-3196 (1987); (composition) Progulske, et al., Inf. Imm. 43(1):166-177 (1984), Keudell, et al., Microbios Lett. 26:23-30 (1984), Mashimo, et al., Microbiol. Immunol. 29(5):395-403 (1985), Tanner, J. Clin. Microbiol. 24(4):562-565 (1986), and Yamazaki, et al., Inf. Imm. 56(1):191-196 (1988); (antigenic structure) Schroter, Ann. Microbiol. (Inst. Pasteur) 125B:59-74 (1974), the disclosures of each of which are hereby incorporated by reference.

LPS is an ideal source of antigens for a variety of reasons. LPS is a cell wall constituent that is present in large amounts in most if not all gram-negative oral microbes, including E. corrodens.

Moreover, LPS appears to exhibit a high degree of resistance, in both its common and variable regions, to change caused by many physico-chemical treatments employed in its extraction and preparation for assay.

Yet another advantage of the use of LPS is that, as an endotoxin as well as cell wall component, some LPS can be expected to be found in an oral sample in an extracellular form as well as in its cellular form see, e.g., C.G. Daly, et al., J. Oral Pathol. 9:1-15 (1980). As a result, there exists a larger detectable pool of LPS than may be the case for antigens that are present only extracellularly or only in the cellular form. The ability to detect simultaneously both forms of LPS as disclosed herein provides, in a sense, the best features of both an endotoxin assay and a whole cell assay.

Antibodies to E. corrodens can be made into a kit to facilitate their use in a variety of immunoassay formats known to those skilled in the art. Preferably the antibodies within a particular kit are monoclonal antibodies specific for a single molecular component of E. corrodens. Most preferably that molecular component is LPS.

The particular immunoassay format in which the presence of E. corrodens is determined is not critical in this invention, so long as the format provides the desired degree of sensitivity and reliability. A number of different types of assays exist having a variety of protocols and labels. For the most part, the commonly available assays for detecting specific determinant sites are competitive protein binding assays, in which antibodies or fragments thereof are employed. As illustrative of the various assays, see U.S. Pat. Nos. 3,654,090, 3,817,837, 4,233,402, 4,275,149 and 4,584,268, the disclosures of which are hereby incorporated by reference. Generally, a reagent solution is formed containing labeled antibody or labeled antigen. The reagent solution can contain, in addition to the labeled component, other additives, such as buffers, e.g.,

phosphate, tris, barbital, or the like, normally at concentrations in the range of about 0.01 to about 10 mM, the concentration being sufficient to maintain a pH in the range of about 6 to about 9, more usually 7 to 8 during the assay. Other additives include preservatives, e.g., sodium azide, inert protein, e.g., serum albumin, sodium chloride, detergents, or the like, which aid in preserving the labeled component, enhancing the formation of the antigen-antibody complex, preventing non-specific binding, or the like.

In such assays the presence and/or amount of complex formed between the antigen and antibody can be determined in a variety of ways depending on the assay format, for instance using immunodiffusion, immunoelectrophoresis, haemagglutination, direct or indirect immunofluorescence, radioimmunoassay, enzyme-linked immunoabsorbent assay, and so on. See, e.g., Roitt, et al., Chapt. 25 "Immunological Tests", in Immunology, Gower Medical Publishing Ltd., New York (1985), the disclosure of which is hereby incorporated by reference. Such methods can be used to determine the presence and/or amount of complex, which in turn can be related to the presence and/or amount of E. corrodens cells and/or antigens, by methods known to those skilled in the art.

The following discussion considers the use of materials such as those described in the assays above in order to perform the method of the present invention. Ideally the assays are dispensed in a fashion that enables their use in a quick and easy fashion by dental personnel, providing usable results without undue time, expense or instrumentation. In a preferred embodiment the method of the present invention can be used in the following manner:

Oral samples can be obtained by any means, and from any source or location that enables one to immunologically react the antigen(s) of interest. Samples need not contain the intact E. corrodens cells themselves, for instance, if the antigen is found extracellularly, or if antigens provided by cell debris are to be detected. Samples can be taken directly from the oral environment, but can also be artificially prepared, e.g., from cultivation or preservation media, from which E. corrodens can be purified or otherwise detected.

In the oral environment samples can be physically obtained, for instance, from saliva, the tongue or tooth surfaces, supragingival plaque, and subgingival plaque.

Studies indicate that the preferred oral sites for sample collection will be gingival crevices and subgingival plaque. Samples can also be assayed in vivo, e.g., by using monoclonal antibodies to detect, localize or otherwise react immunologically with E. corrodens in the oral environment.

Oral samples can be obtained by a variety of means known to the art, including the use of a sterile periodontal scaler, gas-flushed syringe, filter paper strip, glass cuvette or pipette and so on. It is generally not necessary to the practice of this invention to maintain anaerobic, or even sterile conditions since the microbes in samples generally need not be isolated or cultivated. Samples can be analyzed concurrently with sampling or can be stored according to methods known in the art.

Kits prepared using the antibodies of the present invention can include the antibodies alone, or may include a variety of options for packaging of devices and equipment, including filters and reagents, which are currently commercially available and known to those skilled in the art. Useful optional ingredients are second, enzyme-linked antibodies to E. corrodens antibodies of the assay, which will serve as a part of the detection system, as well as buffers, enzyme substrates and the like.

Antibodies labeled with fluorescent compounds or radioisotopes could also be used in a modification of this assay.

The following examples are offered to aid understanding of the present invention and are not to be construed as limiting the scope thereof. Unless otherwise indicated, all percentages are by weight, and the volumes of monoclonal antibodies added represent the volume of culture supernatant of hybridomas grown for at least 3 days at 37°C in HEPES buffered Dulbecco's Modified Eagles Medium supplemented with 10% fetal calf serum and 50 μg/ml gentamycin.

EXAMPLE 1

Production of Monoclonal Antibodies

Five Balb/c Mice (Chas. River, Inc., Portage, WI) were immunized with $4 \times 10^8$ bacteria intraperitoneally at 5 intervals over 4 months. A final intravenous injection was given 3 days prior to the removal of the spleens. The splenic leukocytes from 2 of the immunized mice were fused with the P3X.653 drug-marked myeloma (ATCC CRL 1580, American Type Culture Collection, Rockville, Maryland) and the resulting cell mixture plated into seventeen 96 well microculture plates. After about two weeks small samples of media were removed from all wells exhibiting hybrid growth and tested against a battery of bacteria including: Eikenella corrodens, Bacteroides gingivalis, Bacteroides intermedius, Actinobacillus actinomycetumcommitans, Fusobacterium nucleatum and Streptococcus sanguis. Briefly, 40 $\mu$l of each bacterial suspension was added to the wells of Pandex Epicon Assay Plates (Pandex Laboratories, Mundelien, IL). Twenty $\mu$l of hybridoma culture supernatant was added and incubated for one hour at room temperature. The plates were then washed in an automated immunofluorescent analyzer ("Screen Machine", Pandex Laboratories), and 20 $\mu$l of fluorescein isothiocyanate ("FITC")-labeled goat-anti mouse IgG (KPL Labs, Gaithersburg, MD) was added for an additional 30 minutes. The plates were again washed and the fluorescence bound to the different bacteria was measured by epifluorescence in the above-described analyzer. Wells in which the total fluorescence was 3 to 5 fold higher for E. corrodens as compared with the other bacteria or the negative controls were selected for further expansion and evaluation.

## EXAMPLE 2

## Determination of Species Specified

One hundred clones chosen as described above were assayed for their ability to bind strains of E. corrodens other than the type strain. Samples were obtained from 25 volunteers by swabbing their teeth, gums, and cheeks. The samples were immediately plated on Blood Agar plates and placed in an anaerobic canister for 3 days. The plates were then removed and examined for characteristic corroding colonies according to the method of M. Eiken, Acta. Path. Microbiol. Scand., 43:404-416 (1958), the disclosure of which is hereby incorporated by reference. These colonies were re-streaked and grown on blood agar until pure cultures were obtained, and their identity was confirmed by biochemical tests (Bergy's Manual of Systematic Bacteriology, Jackson, et al., pp. 591-597, Vol. 1, N. R. Krieg and J. G. Holt, eds., Williams & Wilkins, Baltimore, MD (1984)), the disclosure of which is hereby incorporated by reference. Seven such "wild type" strains were identified and were then grown up in 250 ml of "BY" broth prepared as described in Progulske, et al., Inf. Immun. 43:166-177 (1984), the disclosure of which is hereby incorporated by reference. These strains were then used as the solid phase for examination of the 100 hybridoma clones for species specificity. Supernatants from cultures of the 100 clones were each assayed against the seven wild type strains of E. corrodens using the method described in EXAMPLE 1 above. Forty-seven clones were found to react with all 7 wild type strains as well as with the ATCC type strain. Aliquots of these clones were placed in frozen storage, and the remainder of each clone was expanded for further evaluation.

## EXAMPLE 3

## Determination of Cross-reactivity to Human Cells

Ten thousand Hep2 cells (ATCC Accession No. CCL 23, American Type Culture Collection, Rockville, Maryland) were grown in RPMI 1640 with 10% fetal calf serum added (Mediatech, Washington, D.C.) medium in the bottom of microtiter plates for three days at 37° C. The growth medium was removed and the cell monolayer washed three times with phosphate buffered saline ("PBS"). Cells were then fixed with 30% methanol for one hour, washed three more times with PBS to remove the methanol, and stored at -20° C until used. Supernatants of the 47 species specific hybridoma clones described in EXAMPLE 2 were added in duplicate to the wells of the fixed Hep2 plates and incubated for one hour at room temperature. The wells were then washed three times with PBS, and peroxidase-labeled goat anti-mouse IgG was added for an additional hour. The plates were washed and an enzyme substrate solution added. After color development the plates were read in a Dynatech MR600 plate reader at 405 nm. Color development was an indication of antibody binding to the human cells. As can be seen in TABLE 1 below, ten clones were positive for cross-reactivity with the human cells and therefore would not be preferred for diagnostic purposes.

TABLE 1

| Clone | OD405 Human Cells |
|---|---|
| ELK063 | .208 |
| ELK075 | .064 |
| ELK080 | .322 |
| ELK081 | .358 |
| ELK084 | .062 |
| ELK087 | .086 |
| ELK088 | .213 |
| ELK095 | .186 |
| ELK096 | .178 |
| ELK107 | .160 |
| P3X.653 (myeloma)* | .000 |

*Negative Control

## EXAMPLE 4

### Binding to Bacterial Antigens from Growth Medium

Five hundred mls of growth medium in which E. corrodens was grown was centrifuged to remove bacterial cells (15,000 RPM, 30 minutes) and then concentrated over a ultrafiltration membrane having a 10,000 dalton cutoff membrane (Amicon PM10, Amicon, Inc., Danvers, MA). The retentate was then made to 2% with sodium dodecyl sulfate ("SDS") and refiltered over an ultrafiltration membrane having a 100,000 dalton cutoff ("Amicon YM100", Amicon, Inc.). The filtrate of this final filter was immobilized on nitrocellulose and assayed using the 47 species-specific hybridomas described above in EXAMPLE 2. As can be seen in TABLE 2 below, ten clones were found to bind antigens in the filtered growth medium, indicating that the antigen was present in extracellular form.

7

TABLE 2

| Clone | OD405 Culture Medium Extract |
|---|---|
| ELK063 | .031 |
| ELK070 | .205 |
| ELK071 | .035 |
| ELK081 | .056 |
| ELK087 | .029 |
| ELK088 | .143 |
| ELK089 | .088 |
| ELK096 | .046 |
| ELK103 | .047 |
| ELK105 | .233 |
| P3X.653* | .000 |

*Negative Control

EXAMPLE 5

Binding to LPS Coated Latex Beads

Ten mg of LPS isolated from E. corrodens according to the method of Progulske et al., cited previously, was coated onto 20 ml of 2.5% (weight/volume) suspension of latex beads (Fluoricon Polystyrene Assay Particles, #31-000-2, Pandex) for two hours at room temperature. The beads were then washed extensively with PBS to remove any unbound LPS. Fifty μl of supernatant from the 47 species-specific hybridomas described in EXAMPLE 2 were added in triplicate to the wells of Pandex Epicon assay plates. Twenty μl of the LPS-coated beads was automatically added to each well and allowed to incubate for one hour at room temperature. The plates were then automatically washed and 20 μl of FITC goat anti-mouse IgG was added for an additional 15 minutes. The plates were again washed and the fluorescence measured (Relative Fluorescent Units). As seen in TABLE 3 below, three clones were found to bind the E. corrodens LPS-coated beads, indicating that the antigen was LPS.

TABLE 3

| Clone | Relative Fluorescent Units |
|---|---|
| ELK 72 | 2964 |
| ELK 78 | 1529 |
| ELK 91 | 2493 |
| P3X.653 (myeloma)* | 375 |
| PBS* | 219 |
| Mouse sera*** | 3421 |

* negative control
** pooled sera from mice immunized with E. corrodens to produce hybridomas

8

A deposit of the clone having designation ELK 72 was made September 2, 1988, with the American Type Culture Collection and assigned Accession No. HB 9811.

## EXAMPLE 6

### Assay of E. corrodens Species Specific Antibodies Against Selected Type Strains

One hundred thousand of each of the bacteria listed below in TABLE 4 were pipeted into the wells of Pandex Epicon assay plates in triplicate for each of the antibodies tested. Forty microliters of hybridoma supernatant from the listed cell lines was added and incubated for 30 minutes at room temperature. The wells were washed with PBS with 0.05% Tween 20 ("PBS-Tween") and 20 microliters of FITC labeled goat anti-mouse IgG was added. The plates were incubated an additional 30 minutes, washed twice, and the bound fluorescence measured. As can be seen in TABLE 4, only E. corrodens showed antibody binding (i.e., complex formation) significantly above the control background (which was defined as the mean of 3 determinations plus 3 standard deviations (SD) of the mean, and in this EXAMPLE was calculated to be 2300 RFU (mean = 1570, SD = 243)).

TABLE 4

| Bacteria | Relative Fluorescent Units | | |
|---|---|---|---|
| | ELK 72 | ELK 84 | ELK 91 |
| A. actinomycetumcommitans ATCC 29522 | 1267 | 1094 | 1903 |
| A. actinomycetumcommitans ATCC 29523 | 1328 | 1106 | 1294 |
| A. actinomycetumcommitans ATCC 29524 | 973 | 904 | 1194 |
| A. actinomycetumcommitans strain Y4 (L. Wolff*) | 1116 | 1274 | 946 |
| B. gingivalis ATCC 33277 | 1349 | 1333 | 1430 |
| B. intermedius ATCC 25611 | 1092 | 1275 | 1540 |
| B. fragilis ATCC 25285 | 1285 | 993 | ·1392 |
| B. asacchrolyticus ATCC 25260 | 987 | 895 | 1321 |
| B. denticola ATCC 33185 | 1149 | 1552 | 1204 |
| B. melanogenicus ATCC 25845 | 1346 | 1430 | 1145 |
| B. oris ATCC 33573 | 1162 | 1285 | 1090 |
| B. loeschii ATCC 15930 | 1442 | 1194 | 1547 |
| C. ochracea (W. Liljemark*) | 1349 | 1155 | 1268 |
| C. sputigina ATCC 33612 | 1535 | 1208 | 1382 |
| E. corrodens ATCC 23834 | 31496 | 32246 | 30976 |
| E. limosum ATCC 8486 | 926 | 1567 | 1293 |
| F. nucleatum ATCC 25586 | 1237 | 1249 | 1179 |
| H. aphrophlis ATCC 13232 | 1110 | 939 | 911 |
| H. segnis ATCC 7901 | 1614 | 1209 | 829 |
| H. influenzae ATCC 9133 | 1029 | 1452 | 1566 |
| H. parainfluenzae ATCC 7901 | 1340 | 1169 | 1903 |
| T. denticola ATCC 33520 | 2109 | 1543 | 1288 |
| T. denticola ATCC 33521 | 1216 | 1800 | 1490 |
| T. denticola ATCC 35404 | 973 | 1205 | 1694 |
| T. vincentii (R. Johnson*) | 1346 | 1111 | 663 |
| T. phagedenis (R. Johnson*) | 1188 | 1259 | 1307 |
| T. scoliodontum (R. Johnson*) | 1094 | 1190 | 1636 |
| W. recta ATCC 33238 | 1433 | 876 | 1407 |
| S. mutans ATCC 25175 | 872 | 1006 | 1292 |
| S. mutans ATCC 27352 | 934 | 1073 | 1612 |
| S. sanguis ATCC 10556 | 1003 | 1430 | 1397 |

* University of Minnesota

EXAMPLE 7

Assay of E. corrodens in Human Plaque

Twenty plaque samples from healthy volunteers were pooled and diluted 1:4 in sterile PBS. One hundred microliters of the pooled plaque was plated on Eikenella Selective Agar (DiMed, Inc., Minneapolis, MN) and incubated for 48 hours in an anaerobic jar (Baltimore Biological Laboratories, Becton Dickinson, Franklin Lakes, NJ) at 37°C. No growth identifiable as E. corrodens was found, as determined by corrosion of the agar, and the oxidase test described in Bergy's Manual (cited earlier).

This E. corrodens-free plaque was spiked with various concentrations of E. corrodens (ATCC 23834). Fifty microliters of the spiked plaque was combined with 40 microliters of the indicated monoclonal antibody-containing culture supernatant and incubated for 30 minutes at room temperature in a microtiter plate that had been coated with 1 $\mu$g/ml sonicate of E. corrodens and blocked with 0.5% bovine serum albumin ("BSA"). After washing the plates 3 times with PBS-Tween, peroxidase labeled goat anti-mouse IgG was added (5 μg/ml) and the plates incubated an additional 30 minutes. The plates were washed again 3 times with PBS-Tween and peroxidase substrate "ABTS" (2,2'-azinobis(3-ethylbenzthiazoline-sulfonic acid), KPL Laboratories) was added. Color development was measured after 45 minutes using a microplate reader. As seen in TABLE 5, E. corrodens antigens could be detected in the complex chemical environment that typifies plaque, and the amount of color developed was found to be inversely proportional to the amount of E. corrodens added to the plaque sample.

TABLE 5

| Bacteria/Well (Spiked Plaque) | Antibody bound (OD610)* | |
|---|---|---|
| | ELK 72 | ELK 84 |
| Unspiked | 1.090 | 1.160 |
| 10,000 | 1.130 (104)** | 1.080 (93) |
| 100,000 | 0.760 (70) | 0.840 (72) |
| 1,000,000 | 0.025 (2) | 0.220 (19) |
| 10,000,000 | 0.000 (0) | 0.000 (0) |

\* mean of 3 wells for each data point
\*\* % unspiked control

## Claims

1. A method of determining the presence and/or quantitating the amount of Eikenella corrodens in an oral sample comprising the steps of:
    (a) contacting the oral sample with an antibody specific to the species E. corrodens in order to form an immunological complex between antigens of E. corrodens present in the sample and said antibody, and
    (b) determining the presence and/or amount of complex formed.

2. A method according to claim 1 wherein said antigen is lipopolysaccharide.

3. A method according to claim 1 wherein said antibody is a monoclonal antibody.

4. A method according to claim 3 wherein said monoclonal antibody is produced by a hybridoma having ATCC Accession No. HB 9811.

5. A species-specific monoclonal antibody specific for E. corrodens.

6. A monoclonal antibody according to claim 5 wherein said antibody is produced by a hybridoma designated ATCC Accession No. HB 9811.

7. A monoclonal antibody according to claim 5 wherein said antibody is specific for lipopolysaccharide.

8. A kit for the determination of the presence and/or quantitating the amount of E. corrodens in an oral sample comprising a species-specific antibody specific for the species E. corrodens.

9. A kit according to claim 8 wherein said antibody is a monoclonal antibody.

10. A kit according to claim 9 further comprising enzyme-linked antibodies to said species-specific antibody, and substrate for said enzyme of said enzyme-linked antibodies.